(19) Europäisches Patentamt
European Patent Office
Office européen des brevets

(11) Veröffentlichungsnummer: **0 004 889**
**B1**

(12) # EUROPÄISCHE PATENTSCHRIFT

(45) Veröffentlichungstag der Patentschrift: **27.01.82**

(51) Int. Cl.³: **C 07 C 69/07**, C 07 C 33/03, C 07 C 67/04

(21) Anmeldenummer: **79100964.0**

(22) Anmeldetag: **30.03.79**

(54) **Verfahren zur Herstellung terpenoider Formiate und terpenoider Carbinole.**

(30) Priorität: **26.04.78 DE 2818150**

(43) Veröffentlichungstag der Anmeldung: **31.10.79 Patentblatt 79/22**

(45) Bekanntmachung des Hinweises auf die Patenterteilung: **27.01.82 Patentblatt 82/4**

(84) Benannte Vertragsstaaten: **BE CH DE FR GB NL**

(56) Entgegenhaltungen:
**FR-A-1 119 806**
**FR-A-2 302 995**

(73) Patentinhaber: **BASF Aktiengesellschaft, Carl-Bosch-Strasse 38, D-6700 Ludwigshafen (DE)**

(72) Erfinder: **Thoemel, Frank, Dr., Leberstrasse 17, D-6940 Weinheim (DE)**
Erfinder: **Hoffmann, Werner, Dr., Ringstrasse 11 c, D-6701 Neuhofen (DE)**

**EP 0 004 889 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung terpenoider Formiate und terpenoider Carbinole

Die vorliegende Erfindung betrifft ein verbessertes Verfahren zur Herstellung terpenoider Verbindungen der allgemeinen Formel I

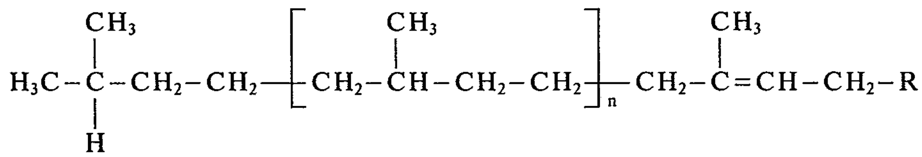 (I)

in der

R für

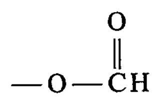

oder —OH und n für 0, 1, 2 oder 3 steht durch Umsetzen der entsprechenden tertiären Vinylcarbinole mit Ameisensäure und gegebenenfalls anschließende hydrolytische Spaltung der Estergruppe.

Die terpenoiden Carbinole der Formel I sind begehrte Verbindungen. Beispielsweise besitzt 6,7-Dihydrogeraniol (I, mit $n = 0$) Bedeutung als Riechstoff und Phytol (I, mit $n = 2$) Bedeutung als Fixateur in der Parfümerie oder auch als Baustein für Naturstoffsynthesen (z. B. Vitamin E aus Phytol und Trimethylhydrochinon).

Bisher wurden die begehrten terpenoiden Carbinole der Formel I im allgemeinen dadurch erhalten, daß man die entsprechenden tertiären Vinylcarbinole der allgemeinen Formel II

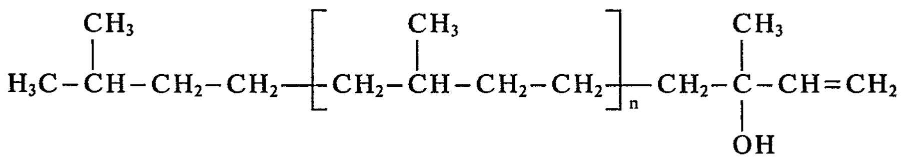 (II)

in der n die oben angegebene Bedeutung hat, in einer unter Umlagerung verlaufenden Reaktion mit Phosphorhalogeniden (B. P. Karrer et al, Helv. Chim. Acta 26 [1943], S. 1741) zu den entsprechenden primären ungesättigten Halogeniden der Formel III

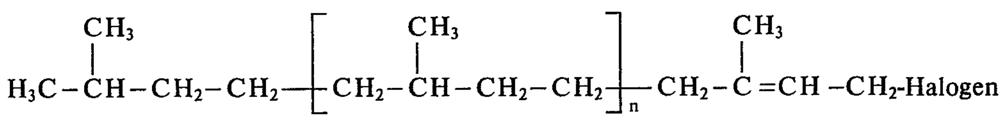 (III)

(Halogen = Chlor oder Brom)

umsetzt und diese in einer weiteren Stufe durch Reaktion mit Natrium- oder Kaliumacetat in die entsprechenden Essigsäureester überführt, aus denen durch Verseifung mit Alkalilauge die terpenoiden Carbinole erhalten werden können. Auf diese Weise wurden z. B. Phytol (P. Karrer et al, Helv. Chim. Acta 26, 1741 [1943]) und Tetrahydrofarnesol (F. G. Fischer und K. Löwenberg, Annalen 475, 183 [1929]) dargestellt.

Es ist ferner bekannt, daß tert.-Vinylcarbinole in Gegenwart von sauren Katalysatoren mit Acetanhydrid oder Essigsäure direkt in die primären ungesättigten Acetate überführt werden können (DE-OS 24 59 546 und DE-PS 80 711). Eigene Versuche zur Umsetzung von Isophytol mit Acetanhydrid in Gegenwart saurer Katalysatoren zeigten jedoch, daß neben dem gewünschten Acetat auch Phytadien entsteht. Infolge seiner emulgierenden Eigenschaften ist das Phytadien auch in geringen Konzentrationen Ursache für die Bildung von schwer zu brechenden Emulsionen bei der üblichen Aufarbeitung des bei diesem Verfahren erhaltenen Reaktionsproduktes.

Läßt man tert.-Vinylcarbinole in Abwesenheit von sauren Katalysatoren mit Acetanhydrid reagieren, so erhält man selbst nach Reaktionszeiten von über 90 Stunden und bei Reaktionstemperaturen von über 100° C die gewünschten Acetate der ungesättigten primären Carbinole nur in Ausbeuten, die 50% d. Th. nicht übersteigen (F. G. Fischer und K. Löwenberg, Annalen 475, 183 [1929]). Unter diesen Reaktionsbedingungen entstehen aus den tert.-Vinylcarbinolen zu einem erheblichen Teil Diolefine. Ausschließlich Kohlenwasserstoffe erhält man nach den Angaben in dieser Arbeit, wenn man Ameisensäure als Reagens verwendet (vgl. loc. cit. S. 192). Es war daher die Aufgabe der Erfindung, ein Verfahren zu entwickeln, mit dessen Hilfe es gelingt, die terpenoiden Carbinole der Formel I aus

den tertiären Vinylcarbinolen der Formel II auf einfache Weise und unter Zuhilfenahme billiger Reaktionshilfsstoffe in guten Ausbeuten herzustellen. In der DE-OS 25 13 198 wird die Umlagerung von Isophytylacetat zu Phytylacetat in Gegenwart von Bis-(benzonitril)-palladiumchlorid beschrieben. Dies Verfahren ist jedoch wegen der Verwendung des teuren Palladiumkomplexes als Katalysator nicht sehr wirtschaftlich. Außerdem gelingt keine direkte Überführung des wohlfeilen Isophytols in das Phytylacetat. Isophytol muß zu diesem Zweck erst verestert werden.

Es wurde nun ein Verfahren zur Herstellung terpenoider Verbindungen der allgemeinen Formel I

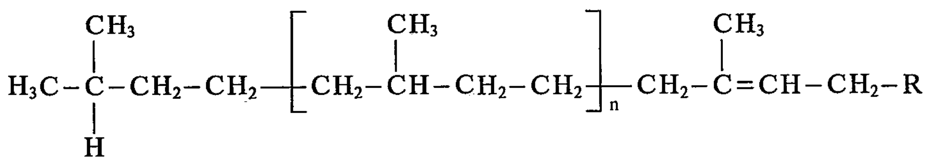

(I)

in der R für —O—CO—H (Ia) oder —OH (Ib) und n für 0, 1, 2 oder 3 steht, gefunden, das dadurch gekennzeichnet ist, daß man

A die entsprechenden tert.-Vinylcarbinole der allgemeinen Formel II

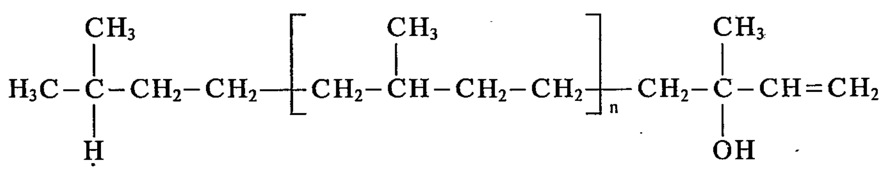

(II)

in der n die oben angegebene Bedeutung hat mit mehr als 2 Mol pro Mol Vinylcarbinol einer etwa 90gewichtsprozentigen wäßrigen Ameisensäure bei Temperaturen von 55 bis 65° C umsetzt und

B für den Fall, daß R für OH steht, die gemäß A enthaltenen terpenoiden Formiate in Gegenwart katalytischer Mengen einer starken Base mit einem niedrigsiedenden Alkohol umestert.

Eine besonders vorteilhafte Ausführungsform des erfindungsgemäßen Verfahrens ist dadurch gekennzeichnet, daß man in Schritt A die tertiären Vinylcarbinole der Formel II mit etwa 10 Mol Ameisensäure pro Mol Vinylcarbinol umsetzt.

Eine weitere vorteilhafte Ausführungsform des erfindungsgemäßen Verfahrens ist dadurch gekennzeichnet, daß man in Schritt A die Reaktion möglichst sofort abbricht, wenn alles eingesetzte Vinylcarbinol umgesetzt ist.

Eine weitere sehr vorteilhafte Ausführungsform des erfindungsgemäßen Verfahrens ist dadurch gekennzeichnet, daß man im Schritt B die erhaltenen terpenoiden Formiate in Gegenwart katalytischer Mengen eines Alkalimethylats mit Methanol unter ständigem Abdestillieren des gebildeten Methylformiats umestert.

Besonders vorteilhaft kann nach dem erfindungsgemäßen Verfahren Phytylformiat hergestellt werden, welches in der Literatur noch nicht beschrieben ist.

Nach dem erfindungsgemäßen Verfahren ist es möglich durch Umsetzen der Vinylcarbinole der Formel II mit Ameisensäure die Formiate der Formel I in nahezu quantitativer Ausbeute zu erhalten, welche ohne besondere Aufarbeitung durch Umesterung in sehr guter Ausbeute in die Carbinole der Formel I überführt werden können.

Das erfindungsgemäße Verfahren eröffnet somit die Möglichkeit, ausgehend von den tertiären Vinylcarbinolen, praktisch in einer Eintopfreaktion die primären Carbinole in Ausbeuten von etwa 70 bis 90% der Theorie zu erhalten.

Dieses äußerst vorteilhafte Ergebnis war sehr überraschend.

Das erfindungsgemäße Verfahren ist deshalb bemerkenswert, weil F. G. Fischer und K. Löwenberg in den Annalen 475(1929), S. 192 beschrieben, daß bei der Umsetzung von Tetrahydronerolidol mit Ameisensäure zum Zwecke der Herstellung von Tetrahydrofarnesol unter allerdings nicht näher angegebenen Verfahrensbedingungen ausschließlich Kohlenwasserstoffe gebildet werden.

Auch die in der Literatur beschriebenen Umsetzungen von tertiären Vinylcarbinolen mit olefinisch ungesättigten Kohlenwasserstoffresten in Gegenwart von Ameisensäure zum Zwecke der Herstellung der entsprechenden primären Carbinolformiate ließen ein solches Ergebnis nicht erwarten, da die Formiate der entsprechenden primären Carbinole in allen Fällen nur in geringen Ausbeuten in einem Produktgemisch von hauptsächlich cyclischen Verbindungen oder von ungesättigten Kohlenwasserstoffen erhalten werden (vgl. J. Bertram und H. Walbaum, J. für prakt. Chem. 45, [1892], 601; O. Zeitschel, Berichte 39, [1906], 1780; L. Ruzicka und E. Carpato, Helv. Chim. Acta 8, [1925], 269).

Es war zwar aus Beispiel 11 der DE-OS 2 509 967 bekannt, 3,6-Dimethyl-hept-1-en-3-ol bei 80° C mit reiner Ameisensäure zum 3,6-Dimethyl-hept-2-en-1-yl-formiat umzusetzen, jedoch waren die hierbei erzielbaren Ausbeuten von 70% für technische Zwecke unbefriedigend. Bei Anwendung der hier beschriebenen Reaktionsbedingungen auf die Umsetzung von Isophytol wurden etwa nur 60%

Phytylformiat im Gemisch mit größeren Mengen an Phytadien erhalten.

Die als Ausgangsstoffe für das erfindungsgemäße Verfahren benötigten tertiären Vinylcarbinole der allgemeinen Formel II sind bekannte Verbindungen, die aus einfachen Ausgangsprodukten auch großtechnisch gut zugänglich sind (vgl. F. G. Fischer und K. Löwenberg, Annalen 475 [1929]; P. Karrer et al, Helv. Chim. Acta 26 [1943], 1741; A. Nürrenbach, Chemie für Labor und Betrieb 28, [1977], 171).

Die erfindungsgemäße Umsetzung der Vinylcarbinole II zu den Formiaten der Formel I gelingt am vorteilhaftesten bei Verwendung einer etwa 90%igen wäßrigen Ameisensäure bei Temperaturen von 55 bis 65° C. Nach dem erfindungsgemäßen Verfahren sind Säuren oder Lewissäuren als Katalysatoren der unter Umlagerung verlaufenden Veresterung nicht erforderlich.

Die Ameisensäure verwendet man im allgemeinen in 2- bis 100fach molarem Überschuß, vorzugsweise in etwa 10fach molarem Überschuß. Bei Verwendung von weniger als 2 Mol Ameisensäure kann die Umsetzung nicht zu Ende verlaufen; mehr als 20 Mol Ameisensäure pro Mol Vinylcarbinol bringen im allgemeinen keinen großen Vorteil mehr.

Verwendet man relativ konzentrierte Ameisensäure, d. h. also beispielsweise Ameisensäure, die weniger als 10 Gew.-% Wasser enthält, so werden in zunehmendem Maße die unerwünschten Diene aus den tertiären Vinylcarbinolen gebildet.

Bei Verwendung einer etwa 90gewichtsprozentigen wäßrigen Ameisensäure bei Temperaturen von 55 bis 65°C sind Reaktionszeiten von etwa 1 bis 2 Stunden vorteilhaft. Mit weiterer Abnahme der Konzentration der Ameisensäure nehmen die zur Umsetzung erforderlichen Reaktionszeiten drastisch zu. Beispielsweise sind bei Umsetzung von Isophytol mit etwa 10 Mol Ameisensäure bei 60°C bei Verwendung einer etwa 83gewichtsprozentigen Ameisensäure bereits Reaktionszeiten von mehr als 8 Stunden und bei Verwendung einer 77gewichtsprozentigen Ameisensäure bereits Reaktionszeiten von etwa 30 Stunden erforderlich. Ein Wassergehalt von mehr als 25 Gew.-% in der Ameisensäure bedingt unvorteilhaft lange Reaktionszeiten und unvollständige Umsätze der Vinylcarbinole II.

Die erfindungsgemäße Umsetzung wird bei 55 bis 65°C durchgeführt. Bei Temperaturen unterhalb 30°C müssen unvorteilhaft lange Reaktionszeiten angewendet werden. Bei höheren Reaktionstemperaturen, d. h. bei Temperaturen oberhalb 70°C treten in verstärktem Maße Nebenprodukte auf. Es werden insbesondere die Diene aus den tert.-Vinylalkoholen gebildet. Die optimalen Reaktionsbedingungen sind für jeden Vertreter der tert.-Vinylalkohole der allgemeinen Formel II innerhalb der angegebenen Grenzen verschieden. Beispielsweise für Isophytol bei Verwendung einer etwa 90gewichtsprozentigen wäßrigen Ameisensäure ist es besonders vorteilhaft das Reaktionsgemisch 2 Stunden lang auf etwa 60°C zu erhitzen. Für die Umsetzung von Tetrahydronerolidol zu Tetrahydrofarnesylformiat ist es besonders vorteilhaft eine etwa 90gewichtsprozentige wäßrige Ameisensäure zu verwenden und das Reaktionsgemisch etwa 1,25 Stunden auf etwa 60° C zu erhitzen.

Die Umsetzung der Reaktionspartner kann in einfacher Weise in Glas- oder Emaillegefäßen durchgeführt werden. Die Reaktionspartner werden zusammengegeben und unter Rühren auf die Reaktionstemperatur gebracht. Nach Reaktionsende trennt sich die wäßrige Ameisensäure als untere Phase rasch und gut vom erhaltenen Formiat ab. Die zurückgewonnene wäßrige Ameisensäure kann man für weitere Versuche verwenden und mehrmals mit konzentrierter Ameisensäure aufkonzentrieren.

Die als Rohprodukte abgetrennten Formiate können durch Erhitzen auf maximal 100°C unter stark vermindertem Druck von der restlichen gelösten Ameisensäure befreit werden. Anschließend können sie durch Destillation gereinigt oder direkt zu den Alkoholen der Formel I weiterverarbeitet werden.

Zu diesem Zwecke versetzt man die gereinigten oder zweckmäßigerweise gleich die rohen, von restlicher Ameisensäure befreiten Formiate der Formel I mit einem Überschuß eines niedrig siedenden Alkohols, d. h. eines $C_1-C_3$-Alkanols, vorzugsweise von Methanol, gibt katalytische Mengen eines basischen Katalysators hinzu und erhitzt das Reaktionsgemisch bis zum Rückfluß. Als basische Katalysatoren können z. B. Alkalialkoholate, Ätznatron oder Kaliumcarbonat dienen. Vorzugsweise verwendet man Alkalialkylate, insbesondere das billige Natriummethylat. Beim Erhitzen der Formiate mit dem niederen Alkohol in Gegenwart der Base destilliert man im allgemeinen langsam den dabei gebildeten Ameisensäureester des niederen Alkohols ab. Als Rückstand verbleibt das Carbinol der allgemeinen Formel I, welches nach dieser Methode in Ausbeuten zwischen 70% und 90% d. Th. erhalten wird. Eigene Versuche zeigen, daß man die Carbinole der allgemeinen Formel I nur in Ausbeuten von etwa 50% gewinnt, wenn man deren Ester statt mit niederen Alkoholen umzuestern, mit Alkalilauge verseift.

Das erfindungsgemäße Verfahren eröffnet somit die Möglichkeit, die begehrten primären Carbinole der Formel I ausgehend von den tertiären Vinylcarbinolen der Formel II praktisch in einer Eintopfreaktion in sehr guten Ausbeuten herzustellen. Die Carbinole der Formel I sind z. T. begehrte Riechstoffe und Fixateure oder als Bausteine für Naturstoffsynthesen von Bedeutung.

## Beispiele 1 bis 8

In einem heizbaren und kühlbaren Reaktionsgefäß aus Glas bzw. Stahl mit Emailleauskleidung, Rührer und kurzer Kolonne wurden Isophytol (Formel III, in der n = 2 ist) und Ameisensäure gemischt

und das Reaktionsgemisch bei der Reaktionstemperatur gerührt.

Angaben über die eingesetzten Mengen an Isophytol und Ameisensäure sowie über die Konzentration der jeweils verwendeten Ameisensäure, über die Reaktionszeit und die Reaktionstemperatur sind in der folgenden Tabelle 1 zusammengestellt.

Anschließend wurde jeweils die sich abtrennende unter Phase (wäßrige Ameisensäure) abgetrennt. Bei den Versuchen 2 bis 4 wurde jeweils die aus dem vorhergehenden Versuch zurückerhaltene wäßrige Ameisensäure als Standardmenge wieder eingesetzt. Das erhaltene rohe Phytylformiat wurde unter auf 20 mbar vermindertem Druck auf 100° C erhitzt, um noch enthaltene Ameisensäure (ca. 1 bis 2 Gew.-%) destillativ zu entfernen. Das so erhaltene Phytylformiat kann direkt umgeestert oder — wenn nötig — durch Destillation gereinigt werden. Der Gehalt an Phytylformiat und Ameisensäure wurde titrimetrisch bestimmt. Alle Reaktionen wurden dünnschichtchromatographisch (Fertigplatten, Kieselgel, Merck; Laufmittel Cyclohexan/Essigsäureäthylester 3 : 1; Sprühmittel $KMnO_4$ in konzentrierter $H_2SO_4$) verfolgt und — soweit möglich — nach vollständigem Umsatz von Isophytol beendet.

Der Siedepunkt des Phytylformiats betrug 160 bis 162° C/3 mbar (physikal. Daten gemäß US-PS 2 638 176: 145 bis 155° C/0,1 mbar $n_D^{25}$ = 1,4540).

Eine Zusammenstellung der erzielten Ergebnisse findet sich in Tabelle 1.

Tabelle 1

| Beispiel | 1 | 2*****) | 3*****) | 4*****) | 5*****) | 6 | 7 | 8*****) |
|---|---|---|---|---|---|---|---|---|
| Isophytol | | | | | | | | |
| [g] | 888 × $10^3$ | 296 | 445 | 296 | 296 | 296 | 296 | 296 |
| [Mol] | 3 × $10^3$ | 1 | 1,5 | 1 | 1 | 1 | 1 | 1 |
| HCOOH | | | | | | | | |
| Menge [g] | 1560 × $10^3$ | 520 | 780 | 520 | 520 | 520 | 460 | 154 |
| Konzentration [Gew.-%] | 90 | 83,1 | 77,5 | 73,4 | 90 | 90 | 100 | 90 |
| Menge [Mol] | 30,5 × $10^3$ | 9,4 | 13,1 | 8,3 | 10 | 10 | 10 | 3 |
| Reaktionstemperatur [°C] | 60 | 60 | 60 | 60 | 40 | 108 | 60 | 60 |
| Reaktionszeit [h] | 2 | 8,5 | 30 | 40**) | 16 | 0,25 | 0,5 | 13****) |
| Phytylformiat: | | | | | | | | |
| Menge an Rohprodukt [g] | 955 × $10^3$ | 307 | 470 | 311 | 301 | 303 | 314 | 300 |
| Gehalt im Rohprodukt [Gew.-%] | 97,2 | 89,0 | 88,5 | 90 | 84,7 | 55 | 86,6 | 72 |
| Ausbeute [% d.Th.] | 98,2 | 84 | 85 | 86 | 79 | 51***) | 84***) | 67 |
| HCOOH*) [g] | 1425 × $10^3$ | 468 | 722 | 515 | 450 | 495 | 397 | 110 |
| Gehalt [Gew.-%] | 83,1 | 77,8 | 73,4 | 68,4 | 83,5 | 84,5 | 94,3 | 75,6 |

*) Zurückgewonnene HCOOH.
**) Der Versuch wurde vorzeitig abgebrochen.
***) Vergleichsbeispiel; der Rest besteht weitgehend aus Phytadien.
****) Es wurde kein vollständiger Umsatz an Isophytol erzielt.
*****) Vergleichsbeispiel.

Beispiel 9

317 g (0,95 Mol) eines gemäß Beispiel 1 erhaltenen rohen Phytylformiats (97%) wurden mit 79 g (100 ml) Methanol und 1 g Natriummethylat versetzt. Aus dem Reaktionsgemisch wurde über eine 30 cm-Glasfüllkörperkolonne bei Normaldruck ein Gemisch aus Methylformiat und Methanol (81 g, Verhältnis 3 : 1) abdestilliert, bis der Siedepunkt auf 64° C angestiegen war (Reaktionszeit 2 Stunden). Als Rückstand verblieben 312 g rohes Phytol, welches über eine Claisenbrücke destilliert wurde. Bei 135 bis 166° C/0,1 mbar gingen 283 g eines Produktes über, welches laut gaschromatographischer Analyse (1 m Versamid-KOH, 250° C) 79,3% Phytol enthält. Die Ausbeute betrug somit 79,8% d. Th.

Beispiel 10

a) Herstellung von Tetrahydrofarnesylformiat

113 g (0,5 Mol) Tetrahydronerolidol (Formel II, in der n = 1 ist) und 260 g (5,1 Mol) 90%ige Ameisensäure wurden analog Beispiel 1 bei 60° C umgesetzt, die Reaktionszeit betrug 1,25 Stunden. Es wurden 230 g einer 82,6gewichtsprozentigen Ameisensäure zurückgewonnen. Das rohe Tetrahydrofarnesylformiat (Formel I, in der n = 1 ist) wurde an einer Claisenbrücke destilliert. Die Ausbeute betrug 113,1 g (entsprechend 90% d. Th.); Kp = 94 bis 96° C/0,1 mbar; $n_D^{25}$ = 1,4999.

b) Herstellung von Tetrahydrofarnesol

50 g (0,22 Mol) des gemäß 10 a) erhaltenen Tetrahydrofarnesylformiats wurden mit 39,5 g (50 ml) Methanol und 1 g Natriummethylat analog Beispiel 9 umgesetzt. Die Ausbeute betrug 34,3 g (entsprechend 80% d. Th.) Tetrahydrofarnesol, Kp = 92° C/0,1 mbar; $n_D^{25}$ = 1,4578 (physikalische Daten gemäß F. G. Fischer und K. Löwenberg: Annalen 475, [1929], S. 193: Kp = 152 bis 156° C/10 mbar; $n_D^{25}$ = 1,4562).

Beispiel 11

a) Herstellung von 3,7,11,15,19-Pentamethyl-2-eicosen-1-yl-formiat

275 g (0,75 Mol) 3,7,11,15,19-Pentamethyl-1-eicosen-3-ol (Formel II, in der n = 3 ist) und 390 g (7,6 Mol) einer 90gewichtsprozentigen Ameisensäure wurden analog Beispiel 1 bei 60° C umgesetzt; die Reaktionszeit betrug 9 Stunden. Es wurden 545 g einer 85,8gewichtsprozentigen Ameisensäure zurückgewonnen. Das rohe 3,7,11,15,19-Pentamethyl-2-eicosen-1-yl-formiat wurde an einer Claisenbrücke destilliert. Die Ausbeute betrug 221 g (entsprechend 74,7% d. Th.); Kp = 176 bis 178° C/0,1 mbar; $n_D^{25}$ = 1,4582.

b) Herstellung von 3,7,11,15,19-Pentamethyl-2-eicosen-1-ol

152 g (0,39 Mol) 3,7,11,15,19-Pentamethyl-2-eicosen-1-yl-formiat wurden mit 79 g (100 ml) Methanol und 2 g Natriummethylat analog Beispiel 9 umgesetzt. Die Ausbeute betrug 86,6 g (entsprechend 61% d. Th.) an 3,7,11,15,19-Pentamethyl-2-eicosen-1-ol, Kp = 177—178° C/0,1 mbar; $n_D^{25}$ = 1,4647.

Beispiel 12 (Vergleichsbeispiel)

a) Herstellung von Dihydrogeranylformiat

312 g (2 Mol) 3,7-Dimethyl-oct-1-en-3-ol (Formel II, in der n = 0 ist) und 1040 g (20,3 Mol) einer 90gewichtsprozentigen Ameisensäure wurden analog Beispiel 1 bei 30° C umgesetzt; die Reaktionszeit betrug 1,5 Stunden. Es wurden 912 g einer 79,8gewichtsprozentigen Ameisensäure zurückgewonnen. Das rohe Dihydrogeranylformiat wurde bei 100° C/15 mbar von der restlichen Ameisensäure befreit. Man erhielt 329 g eines 77,5gewichtsprozentigen Formiats. Die Ausbeute betrug 69,5% d. Th. (Das reine Produkt siedete bei 86 bis 88° C/3,5 mbar, $n_D^{25}$ = 1,4431; physikalische Daten gemäß GB-PS 765 516: Kp = 80° C/3 mbar, $n_D^{25}$ = 1,443).

b) Herstellung von Dihydrogeraniol

329 g (1,39 Mol) des 77,5gewichtsprozentigen Dihydrogeranylformiats wurden mit 237 g (300 ml) Methanol und 5 g Natriummethylat analog Beispiel 9 umgesetzt. Die Ausbeute betrug 219,2 g (entsprechend 67% d. Th.) an Dihydrogeraniol vom Siedepunkt Kp = 70° C/0,2 mbar; $n_D^{25} = 1,4532$ (physikalische Daten gemäß GB-PS 765 516: Kp 118 bis 119° C/20 mbar, $n_D^{25} = 1,4522$).

Beispiel 13 (Vergleichsbeispiel)

Eine Mischung aus 296 g (1 Mol) Isophytol und 460 g (10 Mol) 100%iger Ameisensäure wurde 10 Minuten auf 100° C erhitzt. Es wurden 394 g einer 94,6%igen Ameisensäure zurückgewonnen (untere Phase). Das Produkt der oberen Phase wurde an einer Claisenbrücke bis zum Siedepunkt 29° C/15 mbar andestilliert. Der Rückstand (279 g) enthielt gemäß Titration bzw. dünnschichtchromatographischer Analyse etwa 34,4% Phytylformiat. Dieses Produkt wurde mit 79 g Methanol und 2 g Natriummethylat analog Beispiel 9 verseift und das Rohprodukt destilliert. Man erhielt 181 g eines Gemisches vom Siedepunkt 118 bis 119° C/0,1 mbar, welches laut GC-Analyse 95% Phytadien und 5% Phytol enthielt.

Beispiel 14 (Vergleichsbeispiel)

Eine Mischung aus 296 g (1 Mol) Isophytol und 460 g (10 Mol) 100%iger Ameisensäure ließ man 34 Stunden analog Beispiel 1 bei 5° C reagieren. Es wurden 351 g einer 95%igen Ameisensäure zurückgewonnen. Das rohe Phytylformiat wurde an einer Claisenbrücke bis zum Siedepunkt 32° C/15 mbar andestilliert. Als Rückstand verblieben 306 g eines Produktes, welches gemäß Titration 89,2% Phytylformiat enthielt. Dieses Produkt wurde analog Beispiel 9 mit 79 g Methanol und 2 g Natriummethylat umgesetzt. Die Ausbeute betrug 197 g eines Produktes vom Siedepunkt 136 bis 146° C bei 0,1 mbar, welches laut gaschromatographischer Analyse 81,1% Phytol; 8,6% Isophytol und 9,3% Phytadien enthielt.

Beispiel 15 (Vergleichsbeispiel)

Dünnschichtchromatographische Untersuchung der Umsetzung von Isophytol und Ameisensäure im Molverhältnis 1 : 10.

Eine Mischung von jeweils 1 Mol Isophytol und 10 Mol Ameisensäure der jeweils angegebenen Konzentration wurde auf die angegebene Reaktionstemperatur erhitzt und jeweils nach der angegebenen Reaktionszeit eine kleine Probe des Reaktionsgemisches dünnschichtchromatographisch (Fertigplatten, Kieselgel von Merck; Laufmittel Cyclohexan/Essigsäureäthylester 3 : 1; Sprühmittel $KMnO_4$ in konzentrierter $H_2SO_4$) aufgetrennt und aus der Fleckengröße der gewichtsprozentuale Gehalt abgeschätzt.

A) 70% ige Ameisensäure
Reaktionstemperatur: 100°C

| Reaktionszeit [min] | Isophytol | Phytylformiat [Gew.-%] | Phytadien |
|---|---|---|---|
| 10 | 40 | 40 | 20 |
| 20 | 30 | 50 | 20 |
| 30 | 25 | 55 | 20 |
| 40 | 20 | 60 | 20 |
| 50 | 15 | 65 | 20 |
| 60 | 10 | 70 | 20 |
| 70 | 5 | 75 | 20 |
| 80 | 0 | 80 | 20 |

B) 90% ige wäßrige Ameisensäure
Reaktionstemperatur: 15°C

| Reaktionszeit [Stunden] | Isophytol | Phytylformiat [Gew.-%] | Phytadien |
|---|---|---|---|
| 4 | 95 | 5 | 0 |
| 8 | 90 | 10 | 0 |
| 16 | 85 | 15 | 0 |
| 22 | 80 | 20 | 0 |
| 29 | 75 | 25 | 0 |
| 36 | 70 | 30 | 0 |
| 44 | 65 | 35 | 0 |
| 52 | 60 | 40 | 0 |
| 60 | 60 | 40 | 0 |
| 76 | 60 | 40 | 0 |

C) 96% ige wäßrige Ameisensäure
Reaktionstemperatur: 70°C

| Reaktionszeit [Minuten] | Isophytol | Phytylformiat [Gew.-%] | Phytadien |
|---|---|---|---|
| 10 | 0 | 95 | 5 |
| 20 | 0 | 95 | 5 |
| 30 | 0 | 95 | 5 |
| 40 | 0 | 95 | 5 |
| 50 | 0 | 90 | 10 |
| 60 | 0 | 90 | 10 |

D) 96% ige wäßrige Ameisensäure
Reaktionstemperatur: 80°C

| Reaktionszeit [Minuten] | Isophytol | Phytylformiat [Gew.-%] | Phytadien |
|---|---|---|---|
| 10 | 0 | 85 | 15 |
| 20 | 0 | 80 | 20 |
| 30 | 0 | 80 | 20 |
| 40 | 0 | 75 | 25 |
| 50 | 0 | 75 | 25 |
| 60 | 0 | 70 | 30 |

E) 96% ige Ameisensäure
Reaktionstemperatur: 90°C

| Reaktionszeit [Minuten] | Isophytol | Phytylformiat [Gew.-%] | Phytadien |
|---|---|---|---|
| 10 | 0 | 80 | 20 |
| 20 | 0 | 70 | 30 |
| 30 | 0 | 60 | 40 |
| 40 | 0 | 50 | 50 |
| 50 | 0 | 45 | 55 |
| 60 | 0 | 40 | 60 |

F) 100% ige Ameisensäure
Reaktionstemperatur: 70°C

| Reaktionszeit [Minuten] | Isophytol | Phytylformiat [Gew.-%] | Phytadien |
|---|---|---|---|
| 10 | 0 | 90 | 10 |
| 20 | 0 | 90 | 10 |
| 30 | 0 | 85 | 15 |
| 40 | 0 | 85 | 15 |
| 50 | 0 | 80 | 20 |
| 60 | 0 | 80 | 20 |

G) 100% ige Ameisensäure
Reaktionstemperatur: 80°C

| Reaktionszeit [Minuten] | Isophytol | Phytylformiat [Gew.-%] | Phytadien |
|---|---|---|---|
| 10 | 0 | 85 | 15 |
| 20 | 0 | 80 | 20 |
| 30 | 0 | 70 | 30 |
| 40 | 0 | 60 | 40 |
| 50 | 0 | 50 | 50 |
| 60 | 0 | 50 | 50 |

H) 100%ige Ameisensäure
Reaktionstemperatur: 90°C

| Reaktionszeit [Minuten] | Isophytol | Phytylformiat [Gew.-%] | Phytadien |
|---|---|---|---|
| 10 | 0 | 60 | 40 |
| 20 | 0 | 50 | 50 |
| 30 | 0 | 40 | 60 |
| 40 | 0 | 30 | 70 |
| 50 | 0 | 20 | 80 |
| 60 | 0 | 10 | 90 |

Beispiel 16 (Vergleichsbeispiel)

In einem 3 l-Autoklaven wurde eine Mischung aus 296 g (1 Mol) Isophytol und 920 g (10 Mol) einer 50%igen wäßrigen Ameisensäure auf 150°C erhitzt. Nach den angegebenen Zeitintervallen wurden Proben vom Reaktionsgemisch entnommen und analog Beispiel 15 dünnschichtchromatographisch untersucht.

| Reaktionszeit [Stunden] | Isophytol | Phytylformiat [Gew.-%] | Phytadien |
|---|---|---|---|
| 1 | 70 | 15 | 15 |
| 6 | 30 | 30 | 40 |
| 11 | 5 | 25 | 70 |
| 13 | 0 | 25 | 75 |

**Patentansprüche**

1. Verfahren zur Herstellung terpenoider Verbindungen der allgemeinen Formel I

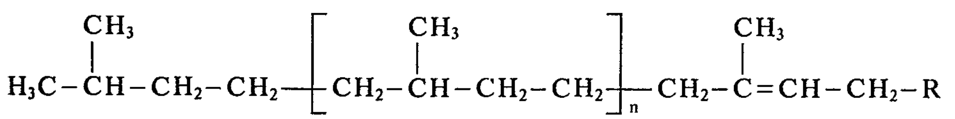

(I)

in der R für —O—CO—H (Ia) oder —OH (Ib) und n für 0, 1, 2 oder 3 steht, dadurch gekennzeichnet, daß man

A die entsprechenden tert.-Vinylcarbinole der allgemeinen Formel II

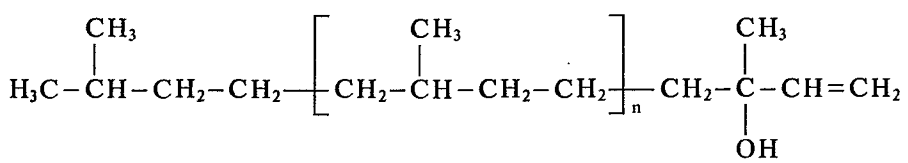

(II)

in der n die oben angegebene Bedeutung hat, mit mehr als 2 Mol pro Mol Vinylcarbinol einer etwa 90gewichtsprozentigen wäßrigen Ameisensäure bei Temperaturen von 55 bis 65° C umsetzt und

B für den Fall, daß R für OH steht, die gemäß A erhaltenen terpenoiden Formiate in Gegenwart katalytischer Mengen einer starken Base mit einem niedrigsiedenden Alkohol umestert.

2. Verfahren zur Herstellung terpenoider Verbindungen der allgemeinen Formel I gemäß Anspruch 1, dadurch gekennzeichnet, daß man in Schritt A die Reaktion abbricht, wenn alles eingesetzte Vinylcarbinol umgesetzt ist.

3. Verfahren zur Herstellung terpenoider Verbindungen der allgemeinen Formel I gemäß Anspruch 1, dadurch gekennzeichnet, daß man im Schritt B die erhaltenen terpenoiden Formiate in Gegenwart katalytischer Mengen eines Alkalimethylats mit Methanol unter ständigem Abdestillieren des gebildeten Methylformiats umestert.

4. Verfahren zur Herstellung terpenoider Verbindungen der allgemeinen Formel I gemäß Anspruch 1, dadurch gekennzeichnet, daß man in Schritt A die tert.-Vinylcarbinole der allgemeinen Formel II mit etwa 10 Mol Ameisensäure pro Mol Vinylcarbinol umsetzt.

5. Verfahren zur Herstellung von Phytylformiat der Formel Ia

$$H_3C-\overset{\overset{\displaystyle CH_3}{|}}{C}H-CH_2-CH_2-\left[CH_2-\overset{\overset{\displaystyle CH_3}{|}}{C}H-CH_2-CH_2\right]_n-CH_2-\overset{\overset{\displaystyle CH_3}{|}}{C}=CH-CH_2-R \qquad (Ia)$$

in der R für —O—CO—H und n für 2 stehen, gemäß Anspruch 1, dadurch gekennzeichnet, daß man Isophytol bei Temperaturen von 55 bis 65°C mit einer etwa 90gewichtsprozentigen wäßrigen Ameisensäure umsetzt.

6. Verfahren zur Herstellung von Phytylformiat der Formel Ia gemäß Anspruch 5, dadurch gekennzeichnet, daß man das Isophytol mit etwa 10 Mol Ameisensäure pro Mol Isophytol umsetzt.

**Claims**

1. A process for the preparation of a terpenoid compound of the general formula I

$$H_3C-\overset{\overset{\displaystyle CH_3}{|}}{C}-CH_2-CH_2-\left[CH_2-\overset{\overset{\displaystyle CH_3}{|}}{C}H-CH_2-CH_2\right]_n-CH_2-\overset{\overset{\displaystyle CH_3}{|}}{C}-CH=CH_2-R \qquad (I)$$

where R is —O—CO—H (Ia) or —OH (Ib) and n is 0, 1, 2 or 3, characterized in that

A. the corresponding tert.-vinylcarbinol of the general formula II

$$H_3C-\overset{\overset{\displaystyle CH_3}{|}}{C}-CH_2-CH_2-\left[CH_2-\overset{\overset{\displaystyle CH_3}{|}}{C}H-CH_2-CH_2\right]_n-CH_2-\underset{\underset{\displaystyle OH}{|}}{\overset{\overset{\displaystyle CH_3}{|}}{C}}-CH=CH_2 \qquad (II)$$

where n has the above meaning is reacted with more than 2 moles, per mole of vinylcarbinol, of aqueous formic acid of about 90 per cent strength by weight at from 55° to 65° C, and

B. if R is OH, the terpenoid formate obtained in A is transesterified with a low-boiling alcohol in the presence of a catalytic amount of a strong base.

2. A process for the preparation of a terpenoid compound of the general formula I, as claimed in claim 1, characterizid in that, in step A, the reaction is stopped when all the vinylcarbinol employed has been converted.

3. A process for the preparation of a terpenoid compound of the general formula I, as claimed in claim 1, characterized in that, in step B, the terpenoid formate obtained is transesterified with methanol in the presence of a catalytic amount of an alkali metal methylate, the resulting methyl formate being distilled off continuously.

4. A process for the preparation of a terpenoid compound of the general formula I, as claimed in claim 1, characterized in that, in step A, the tertiary vinylcarbinol of the general formula II is reacted with about 10 moles of formic acid per mole of vinylcarbinol.

5. A process for the preparation of phytyl formate of the formula Ia

$$H_3C-\overset{\overset{\displaystyle CH_3}{|}}{C}H-CH_2-CH_2-\left[CH_2-\overset{\overset{\displaystyle CH_3}{|}}{C}H-CH_2-CH_2\right]_n-CH_2-\overset{\overset{\displaystyle CH_3}{|}}{C}=CH-CH_2-R \qquad (Ia)$$

where R is —O—CO—H and n is 2, as claimed in claim 1, characterized in that isophytol is reacted with

aqueous formic acid of about 90 per cent strength by weight at from 55° to 65° C.

6. A process for the preparation of phytyl formate of the formula Ia as claimed in claim ! characterized in that isophytol is reacted with about 10 moles of formic acid per mole of isophytol.

**Revendications**

1. Procédé de préparation de composés terpéniques de la formule générale I

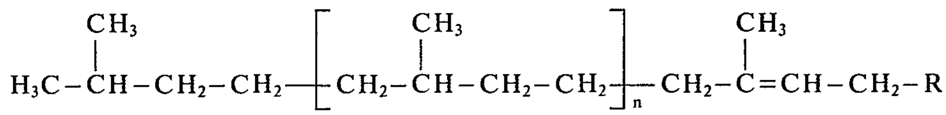 (

dans laquelle R = —O—CO—H (Ia) ou —OH (Ib) et n vaut 0, 1, 2 ou 3, caractérisé en ce que:

A on fait réagir des vinyl-carbinols tertiaires appropriés, de la formule générale II

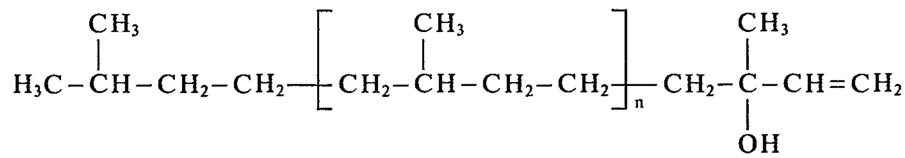 (I

dans laquelle n possède la signification définie, à une température comprise entre 55 et 65° C, ave plus de 2 moles par mole de vinyl-carbinol d'une solution aqueuse à environ 90% en poids d'acid formique, et

B dans le cas où R = OH, on transestérifie les formiates terpéniques obtenus sub A, en présenc d'une proportion catalytique d'une base forte, avec un alcool à bas point d'ébullition.

2. Procédé de préparation de composés terpéniques de la formule générale I suivant l revendication 1, caractérisé en ce que, dans le stade A, la réaction est arrêtée dès que la totalité d vinyl-carbinol mis en oeuvre a réagi.

3. Procédé de préparation de composés terpéniques de la formule générale I suivant l revendication 1, caractérisé en ce que la transestérification des formiates terpéniques obtenus e: réalisée dans le stade B en présence d'une proportion catalytique d'un méthylate de métal alcalin ave le méthanol et séparation continue par distillation du formiate de méthyle formé.

4. Procédé de préparation de composés terpéniques de la formule générale I suivant l revendication 1, caractérisé en ce que, dans le stade A, on fait réagir les vinyl-carbinols tertiaires de l formule générale II avec environ 10 moles d'acide formique par mole de vinyl-carbinol.

5. Procédé de préparation du formiate de phytyle de la formule Ia

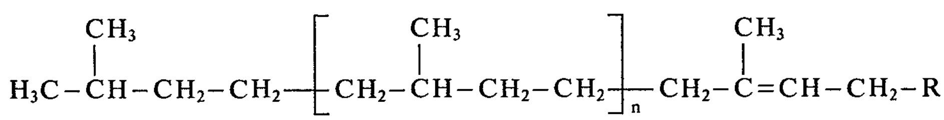 (Ia

dans laquelle R = —O—CO—H et n = 2, suivant la revendication 1, caractérisé en ce que l'on fa réagir de l'isophytol entre 55 et 65° C avec une solution aqueuse à environ 90% en poids d'acid formique.

6. Procédé de préparation du formiate de phytyle de la formule Ia suivant la revendication ! caractérisé en ce que l'on fait réagir l'isophytol avec environ 10 moles d'acide formique par mol d'isophytol.